# EUROPÄISCHE PATENTANMELDUNG

(11) **EP 0 742 212 A1**
(43) Veröffentlichungstag der Anmeldung: **13.11.1996**
(21) Anmeldenummer: 96106718.8
(22) Anmeldetag: 29.04.1996
(51) Int. Cl.: C07D 239/30

(54) **Verfahren zur Herstellung substituierter 2-Fluor-pyrimidine**

(30) Priorität: 11.05.1995 DE 19517186
(71) Anmelder: BAYER AG, 51368 Leverkusen (DE)
(72) Erfinder: Wolters, Erich, Dr., 50939 Köln (DE); Steffan, Guido, Dr., 51519 Odenthal (DE); Klausener, Alexander, Dr., 50670 Köln (DE)

(57) **Zusammenfassung**

Gegebenenfalls substituierte 2-Fluorpyrimidine können durch Reaktion der zugrundeliegenden 2-Amino-pyrimidine mit Diazotierungsmitteln hergestellt werden, wobei Gemische aus HF und H₂O mit 30-70 Gew.-% HF, bezogen auf die Summe von HF und H₂O, als Reaktionsmedium dienen. Das Reaktionsmedium kann auch Verdünnungsmittel und/oder anorganische Salze, bevorzugt Fluoride, enthalten.

## Beschreibung

Gegenstand der Erfindung ist ein neues Verfahren zur Herstellung von 2-Fluor-pyrimidinen durch Umsetzung von 2-Amino-pyrimidinen mit einem Diazotierungsmittel in Fluorwasserstoff-Wasser-Gemischen.

Substituierte 2-Fluor-pyrimidine sind wichtige Ausgangsverbindungen, beispielsweise zur Herstellung von pharmazeutischen und agrochemischen Wirkstoffen, sowie von Farbstoffen. Beispielhaft genannt seien herbizide Wirkstoffe, wie sie etwa in den Patentanmeldungen EP 581 960, EP 514 551 oder EP 547 411 beschrieben werden.

Substituierte 2-Halogen-pyrimidine lassen sich allgemein auf folgenden Wegen herstellen:
1. Durch Diazotierung entsprechend substituierter 2-Amino-pyrimidine mit einem anorganischen Nitrit, wie beispielsweise Natriumnitrit, und anschließende Hydrolyse mit konzentrierter Halogenwasserstoffsäure, analog einem in J. Chem. Soc., C, 1966, 2031 beschriebenen Verfahren. Ein großer Nachteil dieses Verfahrens liegt darin, daß hierbei nur sehr unbefriedigende Ausbeuten erzielt werden. So kann beispielsweise das als Ausgangsverbindung zur Herstellung herbizider Wirkstoffe interessante 2-Chlor-4,6-dimethoxypyrimidin nur in 29 %-iger Ausbeute erhalten werden. Ähnlich schlechte Ergebnisse werden in JP 03 193 766 beschrieben. Die Herstellung von 2-Fluor-4,6-dimethyl-pyrimidin aus dem entsprechenden 2-Amino-pyrimidin gelingt lediglich in 26 %-iger Ausbeute (J. Chem. Soc., Perkin II, 204 (1974)). Die Herstellung des auf Grund seiner gegenüber dem genannten 2-Chlor-4,6-dimethoxy-pyrimidin gesteigerten Reaktivität synthetisch besonders interessanten 2-Fluor-4,6-dimethoxy-pyrimidins durch Diazotierung von 2-Amino-4,6-dimethoxy-pyrimidin ist in der Literatur überhaupt nicht beschrieben.
2. Durch Umsetzung entsprechend substituierter Propan-diimidate oder deren Salze mit Cyanamid in Gegenwart einer Base und nachfolgende Cyclisierung der so erhaltenen entsprechend substituierten Cyan-imidate in Gegenwart von Halogenwasserstoff (EP 582 288). Auf diese Weise ist 2-Chlor-4,6-dimethoxy-pyrimidin in mäßiger Ausbeute zugänglich. Die Ausbeuten im Falle von 2-Brom- 4,6-dimethoxy-pyrimidin bei Anwendung dieses Verfahrens sind sehr klein, und die analoge Herstellung von 2-Fluor-4,6-dimethoxy-pyrimidin ist nicht beschrieben.

Ganz allgemein stellt die Herstellung gegebenenfalls substituierter 2-Halogen-pyrimidine, und unter ihnen insbesondere die Herstellung substituierter 2-Fluor-pyrimidine, ein besonders schwieriges präparatives Problem dar, wie die in der Literatur beschriebenen im allgemeinen geringen Ausbeuten belegen. In besonderem Maße gilt dies, folgt man der veröffentlichten Literatur, für die Synthese gegebenenfalls im Alkylteil substituierter 2-Fluor-4,6-dialkyl-, 2-Fluor-4-alkoxy- und 2-Fluor-4,6-dialkoxy-pyrimidine.

Es bestand daher nach wie vor die Aufgabe, ein technisch durchführbares, zufriedenstellende Reaktionsausbeuten lieferndes Verfahren zur Herstellung substituierter 2-Fluor-pyrimidine, insbesondere im Alkylteil substituierter 2-Fluor-4,6-dialkyl-, 2-Fluor-4-alkoxy- und 2-Fluor-4,6-dialkoxy-pyrimidine, zu entwickeln, das sowohl ökonomischen als auch ökologischen Ansprüchen genügt.

Diese Aufgabe wird durch das erfindungsgemäße Verfahren gelöst.

Die Erfindung betrifft ein Verfahren zur Herstellung von 2-Fluor-pyrimidinen der Formel 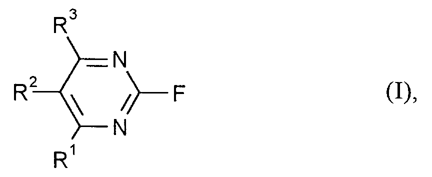 in welcher
- R¹, R² und R³: unabhängig voneinander Wasserstoff gegebenenfalls substituiertes C₁-C₆-Alkyl, C₃-C₈-Cycloalkyl, gegebenenfalls substituiertes Aryl oder Heterocyclyl, Halogen, gegebenenfalls substituiertes C₁-C₆-Alkoxy, C₁-C₆-Alkoxycarbonyl, C₁-C₆-Alkylcarbonyloxy, Cyano, Di-C₁-C₆-alkylamino oder die Gruppe -SOₙ-R⁴, in der n für 0, 1 oder 2 und R⁴ für gegebenenfalls substituiertes C₁-C₆-Alkyl, gegebenenfalls substituiertes C₁-C₆-Alkyloxy, gegebenenfalls substituiertes Aryl, gegebenenfalls substituiertes Aryloxy oder gegebenenfalls substituiertes Arylthio stehen, bedeuten,
das dadurch gekennzeichnet ist, daß man 2-Amino-pyrimidine der Formel 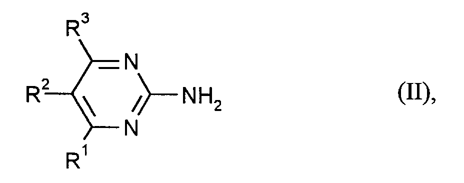 in welcher die Substituenten R¹, R², R³ und R⁴ die oben angegebene Bedeutung besitzen,
in Gemischen aus HF und H₂O mit 30 bis 70 Gew.-% HF, bezogen auf die Summe von HF und H₂O, gegebenenfalls in Gegenwart eines Verdünnungsmittels sowie gegebenenfalls einer oder mehrerer salzförmiger Verbindungen, mit einem Diazotierungsmittel bei -60 bis +40°C zur Reaktion bringt.

Alkyl steht für gegebenenfalls durch Halogen, C₁-C₄-Alkoxy, C₁-C₄-Alkylthio, Halo-C₁-C₄-alkyloxy oder Halo-C₁-C₄-alkylthio substituiertes geradkettiges oder verzweigtes Alkyl mit 1 bis 6 Kohlenstoffatomen, bevorzugt für gegebenenfalls durch Halogen substituiertes Alkyl mit 1 bis 4 Kohlenstoffatomen, und besonders bevorzugt für gegebenenfalls durch Halogen substituiertes Methyl. Insbesondere genannt seien Methyl, Difluormethyl und Trifluormethyl.

Cycloalkyl steht für gesättigtes, gegebenenfalls durch ein bis 3 C₁-C₄-Alkyl oder Halogen substituiertes Cycloalkyl mit 3 bis 8 Kettengliedern, bevorzugt für durch Alkyl substituiertes Cycloalkyl mit 3 bis 6 Kettengliedern, und besonders bevorzugt für Cycloalkyl mit 3 bis 6 Kettengliedern. Insbesondere genannt seien Cyclopropyl und Cyclopentyl.

Gegebenenfalls substituiertes Aryl, Aryloxy sowie Arylthio steht im allgemeinen für gegebenenfalls über Sauerstoff oder Schwefel verknüpftes Phenyl mit gegebenfalls 0 bis 5 weiteren Substituenten, bevorzugt 0 bis 3 weiteren Substituenten und besonders bevorzugt 0 oder einem weiteren Substituenten, wobei die Substituenten aus der Reihe der gegebenenfalls substituierten C₁-C₆-Alkylgruppen, Halogen, gegebenenfalls substituiertem C₁-C₆-Alkoxy, C₁-C₆-Alkoxycarbonyl oder Cyano, bevorzugt aus der Reihe der gegebenenfalls substituierten C₁-C₄-Alkylgruppen, Halogen, C₁-C₄-Alkoxy oder C₁-C₄-Alkoxycarbonyl und besonders bevorzugt aus der Reihe der Alkylgruppen und Halogen stammen und in ortho-, meta- oder para-Stellung zueinander angeordnet sein können.

Gegebenenfalls substituiertes Heterocyclyl steht für einkerniges gegebenenfalls substituiertes gesättigtes, ungesättigtes oder aromatisches Heterocyclyl aus der Reihe 4- bis 6-gliedrigen Heterocyclen mit 1 bis 3 Heteroatomen innerhalb des Ringsystems und bevorzugt für gegebenenfalls substituiertes gesättigtes, ungesättigtes oder aromatisches Heterocyclyl aus der Reihe der 5- bis 6-gliedrigen Heterocyclen mit 1 bis 3 Heteroatomen innerhalb des Ringsystems, wobei als Substituenten Alkyl mit 1 bis 4 Kohlenstoffatomen, Haloalkyl bzw. Halogen, bevorzugt Alkyl mit 1 bis 3 Kohlenstoffatomen, Trifluormethyl und Fluor oder Chlor, in Frage kommen und wobei die genannten Substituenten grundsätzlich beliebige Positionen innerhalb des heterocyclischen Systems einnehmen können. Insbesondere genannt seien 1-, 2- und 3-Pyridinyl, 2-, 4- und 5-Pyrimidinyl, 2- und 3-Furyl, 2- und 3-Thiophenyl, 2-, 4- und 5-Thiazolyl, 2-, 4- und 5-Oxazolyl, 1-Pyrrolyl, 1- und 2-Imidazolyl, 1-Triazolyl, Piperidinyl, Morpholinyl, 4-[2,6-Dimethyl-morpholinyl], 4-[N-Methyl-piperazinyl], Pyrrolidinyl sowie 2- und 3-Oxetanyl.

Halogen steht für Fluor, Chlor, Brom und Iod, bevorzugt für Fluor, Chlor und Brom und besonders bevorzugt für Chlor.

Gegebenenfalls substituiertes Alkoxy steht für gegebenenfalls durch Halogen oder ein weiteres C₁-C₄-Alkyloxy substituiertes Alkoxy mit 1 bis 6 Kohlenstoffatomen, bevorzugt mit 1 bis 4 Kohlenstoffatomen im Alkylteil, wobei Halogen bevorzugt für Fluor und weiteres Alkyloxy bevorzugt für Methoxy steht.

Gegebenenfalls substituiertes Alkoxycarbonyl bzw. Alkylcarbonyloxy hat 1 bis 6 C-Atome, bevorzugt 1 bis 4 Kohlenstoffatomen im Alkylteil und steht besonders bevorzugt für Methoxycarbonyl und Ethoxycarbonyl.

Dialkylamino steht für gleiche oder verschiedene Alkylgruppen mit 1 bis 6, bevorzugt 1 bis 4 Kohlenstoffatomen enthaltendes Dialkylamino.

In bevorzugter Weise werden 2-Amino-pyrimidine der Formel 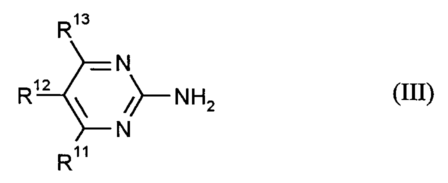 eingesetzt, in der
- R¹¹, R¹² und R¹³: unabhängig voneinander Wasserstoff, gegebenenfalls substituiertes C₁-C₆-Alkyl, C₃-C₆-Cycloalkyl, gegebenenfalls substituiertes Aryl oder Heterocyclyl oder gegebenenfalls substituiertes C₁-C₆-Alkoxy bedeuten.

In besonders bevorzugter Weise werden 2-Amino-pyrimidine der Formel 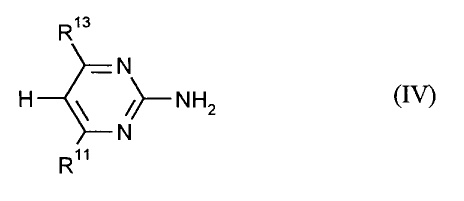 eingesetzt, in der
- R¹¹ und R¹³: unabhängig voneinander Wasserstoff gegebenenfalls substituiertes C₁-C₆-Alkyl, C₃-C₆-Cycloalkyl, gegebenenfalls substituiertes Aryl oder Heterocyclyl oder gegebenenfalls substituiertes C₁-C₆-Alkoxy bedeuten.

In ganz besonders bevorzugter Weise werden 2-Amino-pyrimidine der Formel 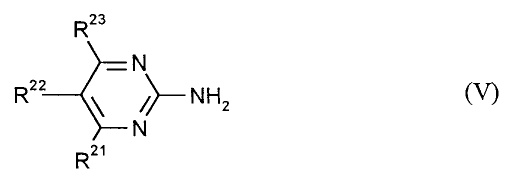 eingesetzt, in der von den Resten
- R²¹, R²² und R²³: einer oder zwei, bevorzugt R²², für Wasserstoff stehen und die verbleibenden Reste unabhängig voneinander Methyl, Ethyl, Methoxy, Ethoxy, 1- bis 3-fach fluoriertes Methyl, 1- bis 5-fach fluoriertes Ethyl, 1- bis 3-fach fluoriertes Methoxy oder 1- bis 5-fach fluoriertes Ethoxy bedeuten.

Insbesondere genannt seien unter den Verbindungen der Formeln (II) bis (V) die in Tabelle 1 aufgeführten:

**Tabelle 1**

| Verbindungen, die auf dem Wege des erfindungsgemäßen Verfahrens besonders gut zugänglich sind | | |
|---|---|---|
| **R**^{**1**} | **R**^{**2**} | **R**^{**3**} |
| CH₃ | H | H |
| OCH₃ | H | H |
| CF₃ | H | H |
| OCF₃ | H | H |
| OCHF₂ | H | H |
| CH₃ | H | CH₃ |
| OCH₃ | H | CH₃ |
| CF₃ | H | CH₃ |
| OCF₃ | H | CH₃ |
| OCHF₂ | H | CH₃ |
| OCH₃ | H | OCH₃ |
| CF₃ | H | OCH₃ |
| OCF₃ | H | OCH₃ |
| OCHF₂ | H | OCH₃ |
| CF₃ | H | CF₃ |
| OCF₃ | H | CF₃ |
| OCHF₂ | H | CF₃ |
| OCF₃ | H | OCF₃ |
| OCHF₂ | H | OCF₃ |
| OCHF₂ | H | OCHF₂ |
| H | OCH₃ | H |

Die Durchführung des erfindungsgemäßen Verfahrens ist dadurch gekennzeichnet, daß man die genannten 2-Amino-pyrimidine in einem Gemisch aus Fluorwasserstoff, Wasser, gegebenenfalls einem oder mehreren weiteren unter den Reaktionsbedingungen stabilen organischen Lösungsmitteln als Verdünnungsmittel sowie gegebenenfalls einem oder mehreren anorganischen Salzen, bevorzugt anorganischen Fluoriden, vorlegt, in dieses Gemisch ein Diazotierungsmittel der Formel

ANO₂ (VI),

in der
- A: für ein Metallion aus der Reihe der Alkalimetalle, für Ammonium oder für geradkettiges oder verzweigtes Alkyl mit 1 bis 6 Kohlenstoffatomen, bevorzugt für ein Metall aus der Reihe der Alkalimetalle und besonders bevorzugt für Natrium oder Kalium steht,
bevorzugt in gelöster Form einträgt und das Reaktionsprodukt nach Beendigung der Reaktion auf an sich bekannte Weise aufarbeitet.

Die als Ausgangsmaterial verwendeten substituierten 2-Amino-pyrimidine sind beispielsweise nach einem in EP 424 849 beschriebenen Verfahren zugänglich.

Die Durchführung des erfindungsgemäßen Verfahrens erfolgt in einem Reaktionsgemisch aus Fluorwasserstoff und Wasser, das, bezogen auf die Gesamtmenge an HF und H₂O, einen HF-Anteil von 30 bis 70 Gew.-%, bevorzugt 40 bis 70 Gew.-% enthält, und dem gegebenenfalls anorganische Salze, bevorzugt anorganische Fluoride, ganz besonders bevorzugt Kaliumhydrogenfluorid (KHF₂), zugesetzt werden, und zwar so, daß sich, bezogen auf das gesamte Reaktionsgemisch, ein Gehalt zwischen 0 Gew.-% und der Sättigungskonzentration dieses anorganischen Salzes bzw. dieser anorganischen Salze ergibt.

Im Einzelfall kann es zweckmäßig sein, diesem Solvatsystem ein oder mehrere weitere organische Lösungsmittel zur Verdünnung beizumischen. Beispielsweise genannt seien Ether, aromatische oder aliphatische Kohlenwasserstoffe, halogenierte Lösungsmittel oder andere unter den Reaktionsbedingungen stabile Solventien. Dabei ist es durchaus möglich, die genannte Reaktion als Zweiphasen-Reaktion durchzuführen. Dies ist in der Regel dann der Fall, wenn das verwendete Verdünnungsmittel sich angesichts größerer Wasseranteile innerhalb des eingesetzten Fluorwasserstoffes entmischt. In bevorzugter Weise wird ohne Lösungsmittel gearbeitet.

Das erfindungsgemäße Verfahren wird im allgemeinen innerhalb eines Temperaturbereiches von +40 bis -60 °C, bevorzugt von 125 bis -40 °C, besonders bevorzugt von +20 bis -20 °C, ganz besonders bevorzugt von +15 bis -20°C durchgeführt.

Der Druck ist für das erfindungsgemäße Verfahren nicht kritisch. Im allgemeinen erfolgt die Durchführung des erfindungsgemäßen Verfahrens in einem Druckbereich von 0,5 bis 1,5 bar, bevorzugt bei Normaldruckbedingungen. Es ist jedoch auch möglich, die Reaktion unter vermindertem Druck oder unter erhöhtem Druck durchzuführen.

Im allgemeinen ist es zweckmäßig, die genannten Nitrite der allgemeinen Formel (VI), soweit es sich um anorganische Stoffe handelt, in Lösung, bevorzugt in wäßriger Lösung, in das Reaktionsgemisch einzutragen. Grundsätzlich ist es aber auch möglich, die genannnten Nitrite in ungelöster Form in das Reaktionsgemisch einzutragen.

Sollten organische Nitrite der Formel (VI) verwendet werden, so können diese in reiner Form, z. B. als gasförmiges Methylnitrit oder als flüssiges Isoamylnitrit eingetragen werden, gegebenenfalls aber auch in verdünnter Form, wie z. B. im Falle des gasförmigen Methylnitrits verdünnt mit Inertgasen wie Stickstoff, Kohlendioxid oder Argon bzw. im Falle flüssiger Alkylnitrite wie Isoamylnitrit verdünnt mit geeigneten unter den Reaktionsbedingungen stabilen organischen Lösungsmitteln.

Im allgemeinen verwendet man stöchiometrisch äquivalente Mengen des Diazotierungsmittels, bezogen auf das eingesetzte, gegebenenfalls substituierte 2-Amino-pyrimidin. Im Einzelfall kann es aber auch zweckmäßig sein, das Diazotierungsmittel im Unterschuß oder Überschuß einzusetzen, etwa im Bereich vom 0,5-fachen bis 3,0-fachen,bevorzugt dem 0,9-fachen bis 2-fachen, besonders bevorzugt dem 1,1-fachen bis 1,8-fachen, bezogen auf die Molmenge des eingesetzten substituierten 2-Amino-pyrimidins.

Im Falle der Durchführung des erfindungsgemäßen Verfahrens unter homogenen Reaktionsbedingungen werden vergleichsweise kurze Reaktionszeiten beobachtet.

Im Falle der Durchführung des erfindungsgemäßen Verfahrens unter zweiphasigen Bedingungen kann die Reaktionsdauer etwas länger sein. Dabei kann es mitunter von großem Vorteil sein, für eine intensive Durchmischung des Reaktionsgemisches durch Einsatz geeigneter Rühraggregate Sorge zu tragen.

Kennzeichnend für das erfindungsgemäße Verfahren und überaus überraschend ist es, daß bei Verwendung ganz bestimmter Mengenverhältnisse von Fluorwasserstoff und Wasser besonders hohe Reaktionsausbeuten gefunden werden. Während z. B. in einem Solvatsystem, das Fluorwasserstoff und Wasser im Gewichtsverhältnis von 20 zu 80 enthält und das zudem mit Kaliumhydrogenfluorid gesättigt wurde, nur sehr geringe Reaktionsausbeuten an z.B. 2-Fluor-4,6-dialkoxy-pyrimidinen erhalten wurden, fanden sich bei Verwendung eines Solvatsystems aus Fluorwasserstoff und Wasser mit einem Gewichtsverhältnis von 40 zu 60 unter ansonsten gleichen Reaktionsbedingungen Reaktionsausbeuten, die im allgemeinen oberhalb von ca. 40 % d. Th. und bei Verwendung eines Gewichtsverhältnisses von 65 zu 35 sogar Reaktionsausbeuten, die oft oberhalb von ca. 70 % d. Th. lagen. Verwendete man jedoch ein Solvatsystem, in dem Fluorwasserstoff und Wasser in einem Verhältnis von mehr als 75 zu 25 vorlag, so wurden wieder deutlich niedrigere Reaktionsausbeuten bei erheblich verlängerten Reaktionszeiten oder unvollständigen Umsätzen beobachtet.

Dieser konzentrationsabhängige Verlauf der Reaktion, bezogen auf die Zusammensetzung des Solvatsystems, der sich in unterschiedlichen Reaktionszeiten und Reaktionsausbeuten niederschlägt, ist überraschend und konnte in der beobachteten Form der Literatur nicht entnommen werden. Des weiteren ist überraschend, daß innerhalb dieses empirisch gefundenen optimalen Konzentrationsbereiches nicht nur eine erhebliche Beschleunigung der betreffenden Umsetzungen beobachtet wird, sondern auch, daß die isolierten Reaktionsprodukte einen erheblich höheren Gehalt aufweisen und sich deutlich leichter aus dem Reaktionsgemisch isolieren lassen.

Das erfindungsgemäße Verfahren wird aus wirtschaftlichen Gründen vorteilhaft so ausgeführt, daß eine möglichst hohe Konzentrationen an Substrat im Solvatsystem vorliegt, soweit dies nicht Ausbeuten und Selektivitäten in unangemessener Weise beeinträchtigt. Die in diesem Sinne optimale Konzentration des Substrats im verwendeten Solvatsystem kann in weiten Grenzen variieren und ist im wesentlichen durch die Eigenschaften des Substrats selbst, durch das gegebenenfalls zusätzlich verwendete organische Lösungsmittel sowie durch die gegebenenfalls zusätzlich im Solvatsystem gelösten anorganischen Salze bestimmt.

Sie liegt im allgemeinen zwischen etwa 5 Gew.-% und 65 Gew.-%, bevorzugt zwischen etwa 10 Gew.-% und 50 Gew.-% und besonders bevorzugt zwischen etwa 10 Gew.-% und 40 Gew.-%, bezogen auf die Summe der Gewichtsmengen der im Solvatsystem enthaltenen Komponenten Fluorwasserstoff und Wasser.

Die Menge des/der gegebenenfalls zusätzlich verwendeten organischen Lösungsmittel(s) kann gleichfalls in weiten Grenzen variiert werden. Sie kann beispielsweise zwischen 0 Vol.-% und 95 Vol.-%, bezogen auf das Gesamtvolumen des übrigen Reaktionsgemisches, betragen.

Die Isolierung der gewünschten substituierten 2-Fluor-pyrimidine der allgemeinen Formel (I) erfolgt nach grundsätzlich bekanntem Verfahren. Es ist jedoch kennzeichnend für das erfindungsgemäße Verfahren, daß besonders saubere Reaktionsprodukte dadurch erhalten werden, daß die rohen Produktgemische zunächst auf einen Fluorwasserstoffgehalt von 10 bis 40 Gew.-% in einem oder mehreren Schritten verdünnt werden, wobei im allgemeinen der größte Anteil des gebildeten Reaktionsproduktes 2-Fluor-pyrimidin in kristalliner Form ausfällt und leicht abgetrennt werden kann. Weitere Mengen des gebildeten Produktes können durch Nachextrahieren des wäßrigen Gemisches mit geeigneten organischen Extraktionsmitteln, z. B. chlorierten Kohlenwasserstoffen, gewonnen werden. Alternativ läßt sich auch das gesamte Reaktionsgemisch auf an sich übliche Weise extraktiv aufarbeiten, wobei die nach Entfernen des Extraktionsmittels erhaltenen rohen Reaktionsprodukte beispielsweise durch Destillation, Umkristallisieren oder Sublimieren gereinigt werden können.

Wird die Reaktion in Gegenwart eines geeigneten organischen Lösungsmittels durchgeführt, so erfolgt bereits auf der Stufe der Verdünnung die Extraktion des Produktes in die organische Phase. Nach Trocknen und Abtrennen des organischen Lösungsmittels bzw. nach Absaugen des kristallin ausgefallenen Reaktionsproduktes kann die Zielverbindung durch übliche Reinigungsverfahren wie z. B. durch Destillation, Kristallisation oder Sublimation, falls gewünscht, weiter aufgereinigt werden.

### Beispiele

### Beispiel 1

In einem 1,5-Liter Teflongefäß, das mit Rührer, Innenthermometer, Tropftrichter und Gasableitungsrohr mit Gasuhr ausgerüstet war, wurden 70,2 g (0,452 mol) 2-Amino-4,6-dimethoxypyrimidin in 450 ml 65 %-iger wäßriger Fluorwasserstoffsäure vorgelegt. Bei einer Temperatur von -10°C ließ man im Verlauf von 2,4 Stunden 93,2 g einer 50%-igen wäßrigen Kaliumnitritlösung (0,547 mol) zutropfen. Während des Zutropfens der Kaliumnitritlösung fand eine rege Stickstoffentwicklung statt. Die Gesamtmenge an entwickeltem Stickstoff betrug ca. 10 l. Dem Reaktionsgemisch wurden nun unter Rühren bei 0°C 300 ml Methylenchlorid und anschließend 300 ml Wasser zugefügt. Die organische Phase wurde abgetrennt, mit Natriumsulfat getrocknet und eingeengt. Es wurden 52,0 g einer weißen kristallinen Masse erhalten, die nach GC, ¹H-NMR und MS zu 98,6 % aus 2-Fluor-4,6-dimethoxypyrimidin bestand. Dies entspricht einer Ausbeute von 71,7 % der theoretischen Ausbeute. Das Produkt kann aus Hexan umkristallisiert oder sublimiert werden und fällt dann in langen weißen Nadeln an.
Fp.: 89-90°C
MS: M⁺=158
¹H-NMR(CDCl₃): 5,95 (d; J_{5,F} 2,67; 5-H) ppm
3,95 (s; (CH₃)₂) ppm

### Beispiel 2

In einem 1,5-Liter Teflongefäß, das mit Rührer, Innenthermometer, Tropftrichter und Gasableitungsrohr mit Gasuhr ausgerüstet war, wurden 70,2 g (0,452 mol) 2-Amino-4,6-dimethoxypyrimidin in 450 ml 40 %-iger wäßriger Fluorwasserstoffsäure vorgelegt. Bei einer Temperatur von -10°C ließ man im Verlauf von 3 Stunden 93,2 g einer 50%-igen wäßrigen Kaliumnitritlösung (0,547 mol) zutropfen. Während des Zutropfens der Kaliumnitritlösung fand eine rege Stickstoffentwicklung statt. Die Gesamtmenge an entwickeltem Stickstoff betrug ca. 10 l. Das ausgefallene, leicht violett gefärbte Produkt wurde abgesaugt, in 200 ml Methylenchlorid aufgenommen, mit Natriumsulfat getrocknet und eingeengt. Es wurden 29,2 g einer leicht violett gefärbten kristallinen Masse erhalten, die nach GC zu 99,0 % aus 2-Fluor-4,6-dimethoxypyrimidin bestand. Dies entspricht einer Ausbeute von 41 % der theoretischen Ausbeute. Die wäßrige Phase wurde fünfmal mit je 50 ml Methylenchlorid extrahiert, die organische Phase mit Natriumsulfat getrocknet und eingeengt. Es wurden 3,5 g einer dunkelviolett gefärbten kristallinen Masse erhalten, die nach GC zu 73,4 % aus 2-Fluor-4,6-dimethoxypyrimidin bestand. Dies entspricht einer zusätzlichen Ausbeute von 3,6 % der theoretischen Ausbeute.

### Beispiel 3

In einem 1,5-Liter Teflongefäß, das mit Rührer, Innenthermometer, Tropftrichter und Gasableitungsrohr mit Gasuhr ausgerüstet war, wurden 70,2 g (0,452 mol) 2-Amino-4,6-dimethoxypyrimidin in 450 ml 65 %-iger wäßriger Fluorwasserstoffsäure und 80,1 g (1,025 mol) Kaliumhydrogenfluorid vorgelegt. Bei einer Temperatur von -10°C ließ man im Verlauf von 2,4 Stunden 93,2 g einer 50 %igen wäßrigen Kaliumnitritlösung (0,547 mol) zutropfen. Während des Zutropfens der Kaliumnitritlösung fand eine rege Stickstoffentwicklung statt. Die Gesamtmenge an entwickeltem Stickstoff betrug ca. 10 l. Dem Reaktionsgemisch wurden nun unter Rühren bei 0°C 300 ml Methylenchlorid und anschließend 300 ml Wasser zugefügt. Die organische Phase wurde abgetrennt, mit Natriumsulfat getrocknet und eingeengt. Es wurden 53,6 g einer weißen kristallinen Masse erhalten, die nach GC zu 98,9 % aus 2-Fluor-4,6-dimethoxypyrimidin bestand. Dies entspricht einer Ausbeute von 74,1 % der theoretischen Ausbeute.

### Vergleichsbeispiel 1

In einem 1,5-Liter Teflongefäß, das mit Rührer, Innenthermometer, Tropftrichter und Gasableitungsrohr mit Gasuhr ausgerüstet war, wurden 70,2 g (0,452 mol) 2-Amino-4,6-dimethoxypyrimidin in 450 ml 75 %-iger wäßriger Fluorwasserstoffsäure vorgelegt. Bei einer Temperatur von -10°C ließ man im Verlauf von 3,2 Stunden 93,2 g einer 50%-igen wäßrigen Kaliumnitritlösung (0,547 mol) zutropfen. Während des Zutropfens der Kaliumnitritlösung fand nur eine geringe Stickstoffentwicklung statt. Die Gesamtmenge an entwickeltem Stickstoff betrug nur ca. 1,2 l. Dem Reaktionsgemisch wurden nun unter Rühren bei 0°C 300 ml Methylenchlorid zugefügt. Die organische Phase wurde abgetrennt, mit Natriumsulfat getrocknet und eingeengt. Es wurden 3,5 g einer weißen kristallinen Masse erhalten, die nach GC zu 98,7 % aus 2-Fluor-4,6-dimethoxypyrimidin bestand. Dies entspricht einer Ausbeute von 4,8 % der theoretischen Ausbeute.

### Vergleichsbeispiel 2

In einem 1,5-Liter Teflongefäß, das mit Rührer, Innenthermometer, Tropftrichter und Gasableitungsrohr mit Gasuhr ausgerüstet war, wurden 70,2 g (0,452 mol) 2-Amino-4,6-dimethoxypyrimidin in 450 ml 20 %-iger wäßriger Fluorwasserstoffsäure vorgelegt. Bei einer Temperatur von -10°C ließ man im Verlauf von 4,5 Stunden 93,2 g einer 50%-igen wäßrigen Kaliumnitritlösung (0,547 mol) zutropfen. Während des Zutropfens der Kaliumnitritlösung fand nur eine sehr geringe Stickstoffentwicklung statt. Die Gesamtmenge an entwickeltem Stickstoff betrug weniger als 1 l. Das ausgefallene, blau gefärbte Produkt, das in Methylenchlorid schlecht löslich war, wurde abgesaugt, mit 200 ml Wasser gewaschen und im Trockenschrank getrocknet. Es wurden 41,4 g einer blau gefärbten kristallinen Masse erhalten, die zu ca. 10 Gew.% aus 2-Fluor-4,6-dimethoxypyrimidin bestand. Dies entspricht einer Ausbeute von ca. 5,7 % der theoretischen Ausbeute.

### Beispiel 4-10

In gleicher Weise, wie im Beispiel 3 beschrieben, wurden aus den entsprechenden 2-Aminopyrimidinen die in der Tabelle 2 aufgeführten Verbindungen hergestellt.

**Tabelle 2**

| Reaktionsprodukt | Ausbeute [%] | ¹H-NMR (CDCl₃) | Fp. [°C] | Kp. [°C/mb] |
|---|---|---|---|---|
| 2-Fluor-pyrimidin | 34,1 | 8,7 ppm, (m), 2H | | |
| | | 7,3 ppm, (m), 1H | | |
| 2-Fluor-4,6-dichlor-pyrimidin | 72,4 | 7,2 ppm, (d) | 38-39 | |
| 2-Fluor-5-brom-pyrimidin | 76,7 | 8,7 mmp (d) | 102 | |
| 2-Fluor-4-methyl-pyrimidin | 44,1 | 8,5 ppm, (m), 1H | | |
| | | 7,1 ppm, (m), 1H | | |
| | | 2,6 ppm, (s), 3H | | |
| 2-Fluor-4,6-dimethyl-pyrimidin | 28,4 | 7,0 ppm, (d), 1H | | |
| | | 2,5 ppm, (s), 6H | | |
| 2-Fluor-4-methoxy-6-methyl-pyrimidin | 45,8 | 6,5 ppm, (d), 1H | 25 | 80/35 |
| | | 4,0 ppm, (s), 3H | | |
| | | 2,4 ppm, (s), 3H | | |

## Patentansprüche

1. Verfahren zur Herstellung von 2-Fluor-pyrimidinen der Formel 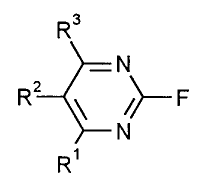 in welcher
R¹, R² und R³ unabhängig voneinander Wasserstoff, gegebenenfalls substituiertes C₁-C₆-Alkyl, C₃-C₈-Cycloalkyl, gegebenenfalls substituiertes Aryl oder Heterocyclyl, Halogen, gegebenenfalls substituiertes C₁-C₆-Alkoxy, C₁-C₆-Alkoxycarbonyl, C₁-C₆-Alkylcarbonyloxy, Cyano, Di-C₁-C₆-alkylamino oder die Gruppe -SOₙ-R⁴, in der n für 0, 1 oder 2 und R⁴ für gegebenenfalls substituiertes C₁-C₆-Alkyl, gegebenenfalls substituiertes C₁-C₆-Alkyloxy, gegebenenfalls substituiertes Aryl, gegebenenfalls substituiertes Aryloxy oder gegebenenfalls substituiertes Arylthio stehen, bedeuten,
dadurch gekennzeichnet, daß man 2-Amino-pyrimidine der Formel 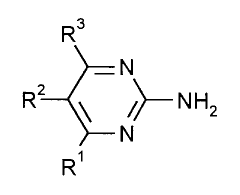 in welcher die Substituenten R¹, R², R³ und R⁴ die oben angegebene Bedeutung besitzen,
in Gemischen aus HF und H₂O mit 30 bis 70 Gew.-% HF, bezogen auf die Summe von HF und H₂O, gegebenenfalls in Gegenwart eines Verdünnungsmittels sowie gegebenenfalls einer oder mehrerer salzförmiger Verbindungen, mit einem Diazotierungsmittel bei -60 bis +40°C zur Reaktion bringt.

2. Verfahren nach Anspruch 1, dadurch gekennzeichnet, daß man 2-Amino-pyrimidine der Formel 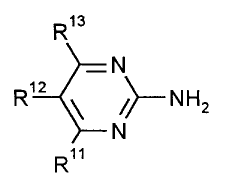 einsetzt, in der
R¹¹, R¹² und R¹³ unabhängig voneinander Wasserstoff, gegebenenfalls substituiertes C₁-C₆-Alkyl, C₃-C₆-Cycloalkyl, gegebenenfalls substituiertes Aryl oder Heterocyclyl oder gegebenenfalls substituiertes C₁-C₆-Alkoxy bedeuten.

3. Verfahren nach Anspruch 2, dadurch gekennzeichnet, daß man 2-Amino-pyrimidine der Formel 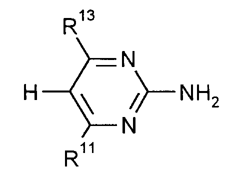 einsetzt, in der
R¹¹ und R¹³ unabhängig voneinander Wasserstoff, gegebenenfalls substituiertes C₁-C₆-Alkyl, C₃-C₆-Cycloalkyl, gegebenenfalls substituiertes Aryl oder Heterocyclyl oder gegebenenfalls substituiertes C₁-C₆-Alkoxy bedeuten.

4. Verfahren nach Anspruch 2, dadurch gekennzeichnet, daß man 2-Amino-pyrimidine der Formel 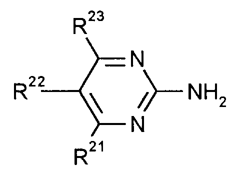 einsetzt, in der von den Resten
R²¹, R²² und R²³ einer oder zwei für Wasserstoff stehen und die verbleibenden Reste unabhängig voneinander Methyl, Ethyl, Methoxy, Ethoxy, 1- bis 3-fach fluoriertes Methyl, 1-bis 5-fach fluoriertes Ethyl, 1- bis 3-fach fluoriertes Methoxy oder 1-bis 5-fach fluoriertes Ethoxy bedeuten.

5. Verfahren nach Anspruch 1, dadurch gekennzeichnet, daß man als Diazotierungsmittels eines der Formel
ANO₂
einsetzt, worin
A für ein Metallion aus der Reihe der Alkalimetalle, für Ammonium oder für geradkettiges oder verzweigtes Alkyl mit 1 bis 6 Kohlenstoffatomen steht.

6. Verfahren nach Anspruch 5, dadurch gekennzeichnet, daß das Diazotierungsmittel im Bereich vom 0,5-fachen bis 3-fachen, bezogen auf die Molmenge des 2-Amino-pyrimidins, eingesetzt wird.

7. Verfahren nach Anspruch 1, dadurch gekennzeichnet, daß das Produktgemisch auf einen HF-Gehalt von 10-40 Gew.-% in einem oder mehreren Schritten verdünnt und dabei auskristallisierendes 2-Fluor-pyrimidin abgetrennt wird.

8. Verfahren nach Anspruch 7, dadurch gekennzeichnet, daß das vom auskristallisierten 2-Fluor-pyrimidin befreite verdünnte Reaktionsgemisch mit Hilfe eines organischen Lösungsmittels extrahiert wird.

9. Verfahren nach Anspruch 8, dadurch gekennzeichnet, daß die Extraktion bei Mitverwendung eines organischen Lösungsmittels simultan zur Verdünnung des ausreagierten Reaktionsgemisches auf einem HF-Gehalt von 10-40 Gew.-% durchgeführt wird.
